# EUROPEAN PATENT APPLICATION

(11) **EP 3 701 994 A1**
(43) Date of publication of application: **02.09.2020**
(21) Application number: 19159752.5
(22) Date of filing: 27.02.2019
(51) Int. Cl.: A61M 25/00, A61M 25/01, A61M 27/00

(54) **URINARY CATHETER ASSEMBLY WITH INTEGRATED HANDLE**

(71) Applicant: Dentsply IH AB, 431 21 Mölndal (SE)
(72) Inventor: ANDERSIN, Per, 435 41 MÖLNLYCKE (SE); LOVMAR, Martin, 431 69 MÖLNDAL (SE)
(74) Representative: AWA Sweden AB

(57) **Abstract**

A urinary catheter assembly comprises a urinary catheter (1) comprising a catheter shaft (1) and a non-insertable rearward part (2), wherein a lumen extends between a drainage opening at or in the vicinity of the insertion end of the catheter shaft and a discharge outlet (23) in the rearward part. Two arms (3) are rotatably connected to the rearward part, wherein the arms are rotatable between a first, storage position, in which the arms are aligned with each other and overlying the catheter shaft, and a second, use position, in which the arms are arranged aligned with each other in a different position, not overlying the catheter shaft.

## Description

### Technical field of the invention

The present invention relates to a urinary catheter assembly. The invention is particularly related to hydrophilic catheters, and specifically to hydrophilic urinary catheters.

### Background

The present invention relates to a urinary catheter assembly. Urinary catheters are commonly used for draining urine from the bladder. Urinary catheters can be of an indwelling type, for long term use, such as days or even weeks, or for intermittent use, whereby the catheters are used for a single draining procedure, typically lasting a few minutes. Intermittent urinary catheters are e.g. used by a large group of persons for self-catheterization, which is a daily-life procedure, taking place several times a day. Typically, catheters for intermittent catheterization are used by patients suffering from urinary retention, due to e. g. spinal cord injury, Multiple Sclerosis or Prosthatic Hyperplasia. Using an intermittent catheter, the bladder may be drained through a natural or artificial urinary canal. Many catheters, such as those for intermittent catheterization, are provided with a hydrophilic coating or the like, providing a smooth and slippery surface when wetted, for safe and comfortable insertion in the urinary canal.

Individuals who suffer from urinary incontinence will normally self-catheterize several times a day. Self-catheterization involves removing the catheter assembly from its package and inserting and advancing the catheter tube through the user's urethra. However, preparation and manipulation of known catheter assemblies is often complicated and tedious. Further, often users of intermittent urinary catheters have limited or diminished dexterity, e.g. as a result of spinal cord injuries. Also, users of intermittent catheters are often required to self-catheterize outside the privacy of the home, such as in public restrooms. Thus, for these and other reasons, it is desirable that the intermittent catheters are provided in discrete packaging that is easy to open and manipulate, which are compact and portable, and wherein the catheter can be deployed and used in a way that alleviates concerns about inadvertent urine leakage or spillage and avoids pain or discomfort to the user.

Many attempts to obtain this have been made in the past. For example, WO 03/002179 proposes a catheter assembly where a handle may be positioned over the catheter shaft in a storage position, and extending away from the catheter shaft in a use position. Similarly, US 2016/0067445 discloses a catheter assembly where a handle is rotatably connected to the rear end of a catheter shaft, so that it can be folded in over the catheter for storage, and folded out for use. US 6653700 discloses a similar arrangement.

Thus, there is still a need for improved urinary catheter assemblies. The assembly should preferably be relatively simple and cost-efficient to produce. Further, the assembly should be easy and intuitive to open and use, even for users with reduced dexterity. The assembly should also preferably be rather small, so that it can easily be carried around by the user in his/her daily life. In particular, it should preferably be compact to minimize the volume during storage, transportation and travel, and should preferably also be compact when it is to be discarded after use.

### Summary of the invention

It is therefore an object of the present invention to provide a urinary catheter assembly which at least alleviates the above-discussed problems.

This object is obtained by means of a urinary catheter assembly in accordance with the appended claims.

According to a first aspect of the invention, there is provided a urinary catheter assembly comprising:
a urinary catheter comprising a catheter shaft and a non-insertable rearward part, wherein a lumen extends between a drainage opening at or in the vicinity of the insertion end of the catheter shaft and a discharge outlet in the rearward part;
wherein two arms are rotatably connected to the rearward part, wherein the arms are rotatable between a first, storage position, in which the arms are aligned with each other and overlying the catheter shaft, and a second, use position, in which the arms are arranged aligned with each other in a different position, not overlying the catheter shaft.

The present invention provides a very compact catheter assembly. In the storage position, the two arms are arranged in parallel with and overlying the catheter shaft. Hereby, the total length of the catheter assembly is only slightly longer than the length of the insertable part of the shaft. In particular, this means that a catheter assembly for female users can be made very compact. The female urethra is normally about 5 cm long, and thus the length of the insertable part of the catheter shaft is preferably 4-10 cm long. Correspondingly, the total length of the catheter assembly for female users may be in the range of 5-15 cm, and preferably in the range 8-12 cm. The arms are preferably slightly longer than the catheter shaft, enabling the entire length of the catheter shaft to be accommodated by the arms in the storage position.

Thus, the catheter assembly provides a compact and neat storage position, when the arms are in the first, storage position. In this position, the catheter assembly could easily be carried around discretely, e.g. in a handbag, in a pocket or the like, without attracting notice. Further, due to the pivotable arms, the catheter assembly can easily be transformed into the use position, in which the arms are aligned in the second, use position, so that the e.g. extends out laterally as a handle, for convenient use. After use, the catheter assembly may be directly discarded. However, it may alternatively be returned to the closed storage position, so that the used catheter assembly can then be carried by the user in the same discrete way as the unopened assembly, and without the risk of spillage etc, to be discarded later.

The urinary catheter assembly is, in the storage position, preferably elongate, and preferably having an essentially rectangular cross-sectional shape. However, other shapes, such as a circular or oval cross-sectional shape are also feasible. The overall appearance of the urinary catheter assembly may e.g. be similar to that of a highlighter marker pen.

The catheter assembly is also relatively simple and cost-effective to produce, since it comprises relatively few, and easily producible parts. For example, the arms and catheter shaft may be produced by extrusion, injection molding or the like. Further, the catheter shaft may be identical or similar to a conventional catheter, and may be produced in conventional ways, and thereafter be attached or included into the assembly.

For use of the catheter assembly, the arms may be pivoted away from each other and into a second aligned position, which is different from the first position. The second aligned position may e.g. be in a position which is directed perpendicular from the direction of the catheter shaft, or a direction which extends away from the rearward end of the catheter, opposite to the direction of the catheter shaft. The preparation process is very simple and intuitive, and resembles the preparation process for a butterfly knife, and can easily be performed also by users have poor dexterity, and inexperienced users.

In the use position, the arms can be used as a handle, for use when the catheter is to be manipulated for insertion into the urethra and subsequent withdrawal when the bladder has been emptied.

Since the arms are arranged in aligned with the catheter shaft in the storage position, relatively long arms can be used, and yet provide a very compact catheter assembly. For example, the arms may have a similar length as the catheter shaft. Hereby, a relatively large handle is provided, which facilitates manipulation of the catheter, in particular for users having poor dexterity. Due to the relatively long length of the arms, the user may also choose whether to grip the handle close to the catheter, or some distance away from the catheter. This is particular advantage, since users may have difficulty in reaching too far down. Further, many users would also prefer to handle the catheter from above, without e.g. having to reach down below the rim of the toilet bowl. Further, the arms make it easy to handle the catheter assembly in a clean and sterile way, e.g. avoiding the need to touch the catheter shaft during insertion.

Since two arms are used, the connection between the catheter shaft and the arms are effectively locked in the use position by simply maintaining the arms together. This requires no substantial force, in particular when relatively long arms are used.

The arms are preferably arranged on different sides of the catheter shaft in the storage position. Hereby, the arms may also, at least partly, form, a package for accommodating the catheter shaft. This package is preferably arranged to maintain the catheter shaft in a sterile condition during storage. Hereby, no additional outer package is needed, which makes both production and handling simpler, and also makes the product more compact.

Thus, in accordance with the present invention, the arms serve two purposes. They function as a protection, or even a package, for maintaining the integrity of at least the insertable section of the catheter, and optionally to also maintain the catheter shaft in a clean and sterile condition during storage, prior to use, and may also function to maintain the catheter in a ready-to-use state, i.e. to function as a fluid impermeable wall enclosing the catheter. When the catheter is a hydrophilic catheter, the case may also accommodate a wetting fluid for activation of the hydrophilic catheter, and preferably for maintaining the catheter continuously wetted and activated during storage. In particular, the wetting fluid may be arranged in direct contact with the catheter during storage. The package can also be resealable, allowing the catheter to be re-accommodated in the housing formed by the arms after use. However, the arms also function as a handle, when in the second disposition, for allowing convenient and effective gripping and handling of the catheter during use, which is of particular importance for users having reduced or poor dexterity. Hereby, a relatively large gripping area is provided, allowing the user to use a multifold various positions and techniques for handling the catheter. A user may e.g. grip the handle provided by the case with the entire hand, in a palmar grip, a position which is very useable in particular for users having poor dexterity. However, the handle may also be gripped by the use of only the fingers, such as two or three fingers, e.g. in a traditional pencil grip, a drumstick grip, a paint brush grip, a cross thumb grip or the like. Thus, the handle is very versatile, allowing it to be gripped in any suitable position.

This function is further enhanced when the arms in the second disposition assumes an angularly displaced position in relation to the longitudinal direction of the catheter shaft, so that it extends laterally from the catheter shaft.

To summarize, the urinary catheter assembly provides many advantages in relation to previously known catheter assemblies, such as a more cost-efficient production and assembling, ease to prepare the catheter for use, including intuitiveness to open, ease of opening, ease of preparing the catheter assembly for use, ease of handling the catheter during catheterization, ease of closing the assembly after use, improved compactness and reduced size, improved discretion, improved portability, improved cleanliness, reduced risk of spillage, improved hygiene, etc.

The catheter is preferably a female urinary catheter. Preferably, the catheter is also a urinary catheter for intermittent, short time use. The term "short term use" indicates a use that is limited in time, and in particular limited to a time period of less than 15 minutes, and preferably less than 10 minutes, and most preferably less than 5 minutes.

The arms are preferably arranged in a first direction in the first, storage position, and in a second direction, being different from said first direction, in the second, use position.

In the first, storage position, the arms preferably form a sealed compartment around said catheter shaft. Hereby, the sealed compartment can maintain the sterility and integrity of the catheter shaft during storage, alleviating the need for further packaging. The sealed compartment may also maintain the catheter in a ready-to-use state, e.g. by maintaining a wetting fluid or moist atmosphere in the compartment during storage. Specifically, it is preferred that at least one of the arms comprises a groove extending in the longitudinal direction of the arm, for accommodation of the catheter shaft in the first, storage position. In a preferred embodiment, both arms are provided with such grooves. It is further preferred that the sealed compartment is fluid impermeable, and preferably at least one of gas impermeable and liquid impermeable. Hereby, vapor and the like is hindered from entering the cavity in which the catheter shaft resides, and in case a wetting fluid is arranged within the cavity, this is hindered from exiting the cavity.

The catheter preferably comprises one or several drainage openings, so-called eyes, arranged at or in the vicinity of the proximal insertion end. However, additionally or alternatively, the proximal end may have a central opening. At least the insertable section of the catheter may be provided with a hydrophilic coating, or in other ways been provided with a hydrophilic surface, which exhibits a lowered friction when wetted.

Further, the non-insertable, rearward part may, at least on a part thereof, have larger cross-sectional dimensions than the catheter shaft. Thus, the rearward part of the catheter may have an increased lateral dimension in at least one direction compared to the catheter shaft.

The catheter may be a hydrophilic catheter, having a hydrophilic surface on at least the insertable section thereof. The hydrophilic surface may be arranged as a hydrophilic coating arranged on a substrate of the catheter, as is per se well known in the art. However, the hydrophilic surface may alternatively be arranged as an integrated part of the catheter, such as an integrated layer, or alternatively, the entire catheter, or part(s) of the catheter, may be made of a hydrophilic material. The hydrophilic surface is preferably arranged to provide low friction when wetted. The hydrophilic surface may be a surface provided with a hydrophilic coating, for example made in accordance with EP 0 093 093 and EP 0 217 771.

The catheter assembly may further comprise a wetting fluid, such as a wetting liquid, for wetting and activation of the hydrophilic surface. The wetting fluid may be arranged in a separate compartment within the assembly, to be released into the compartment housing the catheter shaft at a suitable time, such as immediately prior to use. However, the wetting fluid may also be provided within the same compartment as the insertable section of the catheter, and preferably in direct contact with the insertable section, thereby maintaining the catheter in a wetted, activated state also during storage. This wetted state may be present immediately following closing and sealing of the package, or be obtained after some time of storage. The activated state may thus be provided by pre-wetting of the catheter, prior to arrangement of the catheter in the package, but may alternatively be provided after placement of the catheter in the package. Specifically, wetting of the hydrophilic surface may occur during a period of storage in a sealed container by provision of a humid atmosphere in the package, as is per se known in the art, e.g. by being arranged within a hydration element, such as a chamber or sachet which is liquid impermeable and vapor permeable.

Since the hydrophilic surface is then maintained in a wetted state during storage, the medical device is immediately ready-to-use upon removal from the package, and need not be wetted or treated in any way prior to use.

The package is preferably formed by the arms, and is preferably impermeable to the wetting fluid, and preferably made of a gas impermeable material. This ensures that moisture does not penetrate out from the package during storage, and enhances the shelf-life of the product.

The urinary catheter assembly of any one of the preceding claims, wherein the arms when arranged in the second, use position are arranged at an angle in relation to the longitudinal direction of the shaft, said angle being in the range of 80-180 degrees, and preferably in the range of 85-140 degrees, and most preferably in the range of 90-120 degrees. In one embodiment, the angular position is about 90 degrees. Alternatively, the arms, when arranged in the second, use position, are arranged at an angle in relation to the direction of the shaft, said angle being in the range of 80-120 degrees, and preferably in the range of 85-115 degrees, and most preferably in the range of 90-110 degrees. Alternatively, the arms, when arranged in the second, use position, are arranged at an angle in relation to the direction of the shaft, said angle being in the range of 140-180 degrees, and preferably in the range of 150-180 degrees, and most preferably in the range of 160-180 degrees.

Depending on the angle between the arms and the catheter shaft in the second, use position, it is in many embodiments, such as for a 90 degrees embodiment, possible to use the surface of the arms facing the catheter shaft in the use position as a mirror. To this end, one or both of the arm surfaces turned towards the catheter shaft may be reflective and function as mirrors. Hereby, the user gets further assistance during maneuvering of the catheter, which is of particular use for female users during insertion of the catheter into the urethra.

The arms are preferably rotatable or pivotable in opposite directions when rotated between the first, storage position and the second, use position.

The urinary catheter assembly preferably further comprises a valve, the valve closing the lumen when the arms are in the first, storage position, and the valve opening the lumen when the arms are in the second, use position. By this arrangement, no additional closing of the lumen is necessary for closing the package. Thus, rotation of the arms will in this case both expose the catheter shaft for use, prepare the arms for use as a handle, and open up the lumen for urine drainage, all in one action. However, alternatively, an additional closure, such as a removable cap, a tape, a membrane, or the like, may be used for closing the drainage opening.

The arms may be rotatably connected to the rearward part of the catheter in various ways. In one embodiment, each of the arms comprise a stub which is inserted into a corresponding hole or socket in the rearward part of the catheter. The stub hereby forms the rotation axis around which the arm is rotatable. In case the arms, when in the use position, are to extend in a direction being opposite to the catheter shaft, the rotation axes are preferably arranged essentially perpendicular to the longitudinal direction of the catheter shaft. However, if the arms, in the use position, are to extend at an angle towards the longitudinal direction of the catheter shaft, such as perpendicular to this, the stubs and the openings/sockets are preferably arranged at a non-perpendicular angle towards the longitudinal direction of the catheter shaft, such as at 45 degrees angle to form a perpendicularly extending handle.

In one embodiment, the lumen extends only between the drainage openings, the eyelets, at or in the vicinity of the insertion end, to the discharge opening at the rearward part. Thus, drained urine will then be discharged from the discharge opening into a toilet bowl, or into an external tube or the like connected to the discharge opening, e.g. for draining urine into an external urine collection bag. However, in an alternative embodiment, at least one of the arms may further be provided with an additional lumen which is arranged to be in fluid communication with the lumen of the catheter shaft when the arms are arranged in the second, use position. Hereby, the urine will flow from the eyelets, through the first lumen to the discharge opening at the rearward part, and then continue in the additional lumen in one or both of the arms, to one or two second discharge openings. Hereby, the arms and the additional lumen(s) provide an extension of the urine flow path.

These and other aspects of the invention will be apparent from and elucidated with reference to the embodiments described hereinafter.

### Brief description of the drawings

For exemplifying purposes, the invention will be described in closer detail in the following with reference to embodiments thereof illustrated in the attached drawings, wherein:
Figs 1A and 1B are perspective views of a urinary catheter assembly in accordance with an embodiment of the present invention in storage position, shown in Fig 1A, and a use position, shown in Fig 1B;
Fig 2A and 2B are exploded perspective views of the urinary catheter assembly of Fig 1, where Fig 2A shows the assembly from slightly above, whereas Fig 2B shows the assembly from slightly below; and
Fig 3A and 3B shows detailed views of a valve function provided in the rotatable connection between the arms and the rearward part of the catheter, where Fig 3A illustrates a closed valve and Fig 3B illustrates an open valve.

### Detailed description of preferred embodiments

In the following detailed description preferred embodiments of the invention will be described. However, it is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations of the invention, e.g. the length of the medical device, etc.

The urinary catheter assembly disclosed in relation to the illustrative example has an elongate shape, and having a generally rectangular cross-section. However, as discussed in the foregoing the assembly may also have other shapes, such as being of a circular or oval shape.

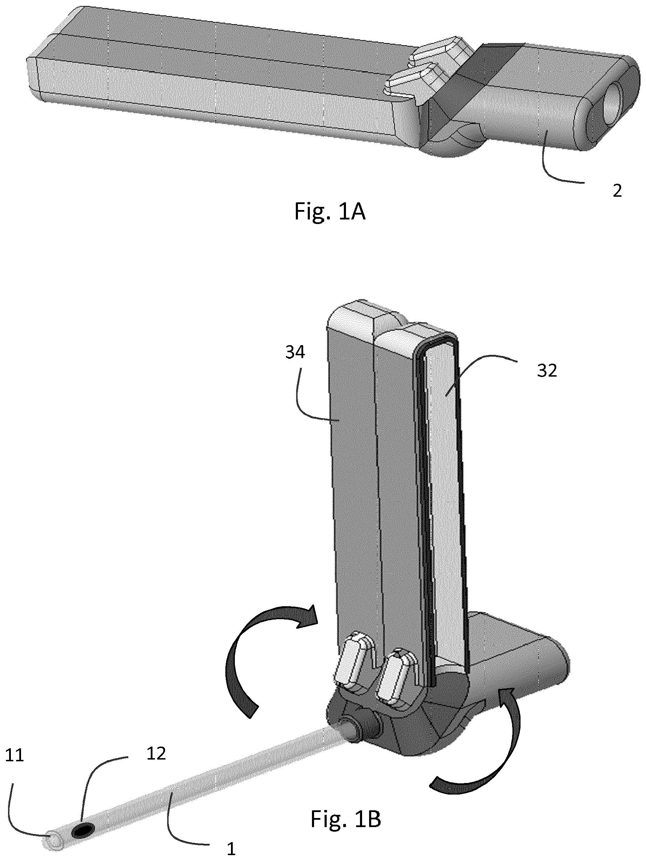

The catheter assembly as illustrated in Figs. 1-3 comprises a catheter 1 having an insertable section, forming a catheter shaft 1, and a non-insertable, rearward part 2, which may alternatively be referred to as a connector or connector part.

At least a part of the catheter shaft 1 forms an insertable length to be inserted through a urethra of the user.

The catheter shaft comprises an insertion tip 11, which may be a closed, rounded end. Further the catheter shaft may comprise inlet openings 12, so called catheter eyes or eyelets, leading into a lumen extending through the catheter, and into a discharge outlet 23 arranged at the rearward end of the non-insertable section.

The catheter shaft 1 may comprise a hydrophilic surface, and form a hydrophilic catheter, as is per se well known in the art. The hydrophilic surface may be in the form of a hydrophilic surface coating, for example PVP, and which provides a low-friction surface when wetted with a wetting fluid. Typically, the insertable length is within 50-140 mm for a female patient and 200-350 mm for a male patient. Even though PVP is the preferred hydrophilic material, other hydrophilic materials may be used, such as hydrophilic polymers selected from polyvinyl compounds, polysaccharides, polyurethanes, polyacrylates or copolymers of vinyl compounds and acrylates or anhydrides, especially polyethyleneoxide, polyvinyl-pyrrolidone, heparin, dextran, xanthan gum, polyvinyl alcohol, hydroxy propyl cellulose, methyl cellulose, copolymer of vinylpyrrolidone and hydroxy ethylmethyl acrylate or copolymer of polymethylvinyl ether and maleinic acid anyhydride. However, instead of a hydrophilic surface coating, the entire insertable section of the catheter may be formed of a hydrophilic material.

However, alternatively the catheter may be non-hydrophilic, and may e.g. be lubricated with gel prior to insertion.

The catheter shaft can be formed in any conventional way, as is per se known in the art, such as by extrusion and thermoforming, or by injection molding.

The non-insertable, rearward part 2 preferably has a larger diameter than the catheter shaft 1, and may also be made of a more rigid material. In the illustrative example, the rearward part 2 is formed by two separate parts, 21 and 22, where part 21 is the most rearward part, and the part 22 is an intermediate part, arranged between the catheter shaft and the most rearward part 21.

The intermediate part 21 comprises a protruding tubular member 24, in which the catheter shaft may be mounted and fixated. The catheter shaft may e.g. be fixated in the tube by adhesive, welding or the like.

In the illustrative example, the fluid path formed by the lumen in the catheter shaft continues into the interface between the two parts 21 and 22, where the fluid path is branched and bifurcated into two parallel fluid paths, and continues into the interior of the most rearward part 21, where the two fluid paths are rejoined and continues to the discharge opening 23. This branching of the fluid path enables a valve functionality which is controlled by the arms, as will be discussed in more detail in the following. However, alternatively, the fluid path may extend only on one side, to provide a single valve instead. Further, it is also feasible to provide a straight lumen and fluid path also in the rearward part 2, leading directly and in a straight line from the end of the catheter shaft and to the discharge end. In such an embodiment, it may be advantageous to provide a cap or other form of closure to close the discharge end prior to use. If a straight lumen is provided in the rearward part, the rearward part may alternatively be formed as a single, monolithic element, instead of the two elements used in the illustrative embodiment.

The rearward part may have a somewhat cuboidal form, at least in the most rearward section, and preferably with softly rounded edges and sides.

The discharge opening 23 may be internally flared or funnel-shaped, and may be arranged to be connected to a tapered connection part of a urine collection bag or the like. It may also be connected to an extension tube, or be used for direct discharge of the urine into a toilet bowl or the like.

The two elements 21 and 22 forming the rearward part are preferably made of the same material, and the parts may be injection molded, and later assembled together. The elements may be connected to each other by a friction fit, a snap-lock arrangement or the like, but may alternatively or additionally be connected by adhesive, welding or the like. In the illustrative example, element 21 is provided with a groove 25 encircling the rim of the interface, and element 22 is provided with a correspondingly shaped ridge 26, allowing the parts to be press fit together. The fixed connection can also be obtained already during manufacturing of the elements, e.g. by use of 2-component (2k) injection molding.

The element 22 is further provided with two sockets or openings 27, arranged on each side of the protruding tubular member 24.

Two arms 3 are further provided. The arms are preferably essentially identical, but mirror inverted. Each arm comprises a stub 31 to be accommodated by the sockets 27. The stubs 31 and sockets 27 form a rotational joint between the arms and the catheter, allowing the arms to be rotated around rotational axes formed by the stubs. In the illustrative example, the longitudinal direction of the stubs and the sockets are at a non-perpendicular angle to the longitudinal direction of the catheter shaft, such as 45 degrees. To this end, the surface of the element 22 facing the catheter, and in which the sockets are provided, may also be slanted, so that it extends generally perpendicular to the rotational axes.

The stubs 31 and the sockets may be connected together in various ways, such as being press fitted together. The stubs may also have some form of outward protrusion close to the insertion ends, to form a snap-lock. The protrusion may e.g. be in the form of a circular protrusion, as shown in the illustrative example.

In a first position, the arms are aligned with each other, and are arranged aligned with and overlying the catheter shaft, as shown in Fig. 1A. At least one of the arms, and preferably both, has a groove 32 forming a through. Together, the two arms hereby, when brought together, forms a cavity for accommodation of the catheter shaft. The sides of the groove(s) are preferably of equal height around the groove, apart from a small recess for accommodation of the protruding tubular member 24 extending out from the rearward part, and in which the catheter shaft is connected. Hereby, when closed, the cavity forms a closed compartment for the catheter shaft. This compartment is preferably able to maintain sterility in the cavity, and is further preferably arranged to provide a gas and/or liquid impermeable barrier to the cavity.

To ensure sterility and gas impermeability, and to maintain the arms together in the storage position before an intended use, the abutting surfaces of the arms may be provided with an adhesive or sealant 33, such as a 2k soft sealing material. However, the connection between the arms is preferably relatively weak, allowing the arms to be easily disconnected from each other at will, when the catheter assembly is about to be used.

When the catheter assembly is to be used, the arms are disconnected from each other, by pulling them outwardly, away from each other. Each arm is then pivoted around its rotational axis formed by the stub and socket interface, until the arms again meet in a second aligned position. This second position is different from the first position. Such a second, use position is illustrated in Fig. 1B. In the illustrative example, due to the angles of the rotational axes, the arms here extend laterally from the catheter, generally perpendicularly to the longitudinal direction of the catheter shaft. However, it should be appreciated by the skilled reader, by using different angles of the rotational axes, different angular orientations of the arms in the second position will be obtained. For example, if the rotational axes are instead arranged essentially perpendicular to the longitudinal direction of the catheter shaft, the arms will in the second position instead extend away from the catheter shaft in a direction opposite to the longitudinal direction of the catheter shaft.

In the second, use position, the arms are again aligned, and forms a handle which may be used for manipulation of the catheter. By holding the arms together, a very firm and stable connection between the handle and the catheter is obtained, without the need of any significant force.

In order to further assist the user during the catheterization, and in particular to aid in finding the urethral meatus for catheter insertion into the urethra, the surfaces of the arms facing the catheter shaft in the use position may be formed as mirror surfaces 34, e.g. by attachment of a mirroring layer on these surfaces. This is particularly useful for female users, and allows the user to see both the urethral meatus and the catheter tip during the catheterization.

As already discussed, the arms may together form a sealed cavity when arranged in the first, storage position. However, to complete the closing of the cavity, the fluid path leading between the discharge opening 23 and the eyelets 12 may also need to be closed. This can be obtained in various ways, such as by providing a detachable cap in the discharge opening or the like. However, in a preferred embodiment, a valve is provided, which automatically opens when the arms are moved from the storage position to the use position. There are various ways for realizing such valves. One possible embodiment for such valves will be discussed in more detail in the following.

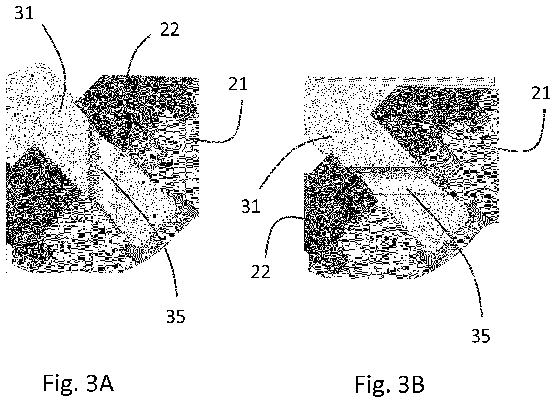

In this illustrated embodiment, the stubs 31 each comprises a channel 35 leading across the diameter of the stub, and in a somewhat slanted disposition, so that the channel openings occur at different heights at the different sides. In the storage position, illustrated in more detail in Fig. 3A, the channel 35 is arranged so that the channel openings are not aligned with the openings of the flow path leading through the rearward part elements 21 and 22. Hereby, the flow path is closed, and the cavity housing the catheter shaft is sealed. When the arms are rotated to the use position, the stub is rotated 180 degrees within the socket, whereby the channel openings switch places within the socket. In this position, the channel openings are instead aligned with the flow path openings, thereby opening the flow path, and allowing urine to be drained through the flow path. This position is illustrated in Fig. 3B.

However, alternative valve arrangements are also feasible. For example, only one of the stubs may form a valve. It is also possible to connect one or both stubs to a separate valve, and to transfer a movement of the stubs to the valve for opening and closing.

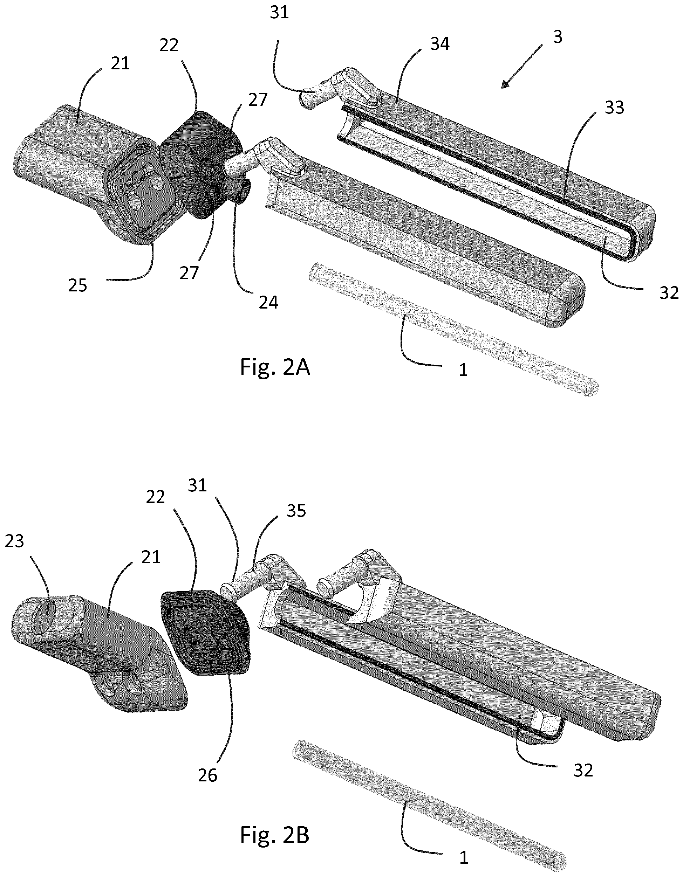

For producing and assembling the urinary catheter assembly, the catheter shaft 1, the rearward part elements 21 and 22, and the arms 3 may each be produced and provided separately, as shown in the exploded view in Figs 2A and 2B.

The catheter shaft can e.g. be produced in a conventional way, using conventional manufacturing equipment. The catheter can be produced by any biocompatible polymeric material having sufficient stiffness and flexibility, as is per se well known in the art.

The rearward part elements may be produced by the same material as in conventional catheter connectors. These elements are preferably injection molded, and may be injection molded separately, and then fitted together, or be produced and assembled simultaneously, e.g. by 2k injection molding.

The arms may be made of the same material as the rearward part, or of a somewhat harder material. For example, the arms can be made of relatively hard plastic material, such as HDPE, nylon or polypropylene. The arms are also preferably produced by injection molding. The arms and/or rearward part of the catheter may further be produced by a non-transparent and opaque material, such that the contents cannot be easily identified.

For assembling the urinary catheter assembly, the catheter shaft is mounted to the rearward part, before or after connecting the two rearward part elements together. Thereafter, the arms are connected to the rearward part, and then connected together to accommodate the catheter in the cavity formed therein.

However, as an alternative, it may also be possible to assemble two or more of the components already during manufacturing of the parts, e.g. by use of two-shot molding processes or the like. Further, the assembling steps may also be performed in a different order.

The assembly is then sterilized and ready for use.

In case a hydrophilic catheter is used, the urinary catheter assembly may also comprise a wetting fluid. However, the wetting fluid for activation of the catheter need not be provided within the assembly. Instead, a wetting fluid may be poured into the assembly after opening of the discharge opening, for wetting of the catheter while it still remains in the cavity formed by the arms. In some occasions, the catheter may even be exposed, by bringing the arms to the use position, prior to wetting, for wetting of the catheter, e.g. in a different container, even though this is normally not preferred.

However, preferably the wetting fluid is arranged within the assembly, so that the hydrophilic surface of the catheter can be activated even before opening of the assembly. In one embodiment, the wetting fluid is arranged separated from the catheter shaft, in a wetting fluid container (not shown), such as a pouch or a sachet, or in a separate compartment integrated within the assembly. The wetting fluid container may be openable by means of e.g. exerting a pressure to the container, whereby the wetting fluid is released into the package, thereby wetting the hydrophilic surface of the catheter. The wetting fluid container may also be arranged to open automatically when the catheter assembly is opened, e.g. by the opening of a cap or the rotation of the arms. In another embodiment, the wetting fluid is arranged directly in the cavity accommodating the catheter shaft, so that the hydrophilic surface of the catheter is in direct contact with the wetting fluid during storage, and thereby is maintained in an activated, ready-to-use state.

The wetting fluid is preferably a liquid, and most preferably an aqueous liquid, such as water or saline. However, the wetting fluid may also be a gas, such as vapor, providing a moist atmosphere in the package sufficient for activation of the hydrophilic surface. Thus, the wetting fluid may be any fluid, gas or liquid, that wets/activates a hydrophilic surface of the catheter.

In case a non-hydrophilic catheter is used, the urinary catheter assembly may comprise a supply of lubricant, such as a compartment arranged overlying the catheter shaft, so that the shaft is lubricated while being extracted out from the assembly.

Specific embodiments of the invention have now been described. However, several alternatives are possible, as would be apparent for someone skilled in the art. For example, although the wetting fluid in the described embodiments is arranged in direct contact with the catheter, but may alternatively be arranged separated from the catheter, in a wetting fluid container. Further, it is possible to use many different types of valve solutions for opening and closing the lumen. Further, the rotational axes of the arms may be arranged in many different angles in relation to the catheter, providing various positions for the arms in the use position. Such and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements. Further, a single unit may perform the functions of several means recited in the claims.

## Claims

1. A urinary catheter assembly comprising:
a urinary catheter comprising a catheter shaft and a non-insertable rearward part, wherein a lumen extends between a drainage opening at or in the vicinity of the insertion end of the catheter shaft and a discharge outlet in the rearward part;
wherein two arms are rotatably connected to the rearward part, wherein the arms are rotatable between a first, storage position, in which the arms are aligned with each other and overlying the catheter shaft, and a second, use position, in which the arms are arranged aligned with each other in a different position, not overlying the catheter shaft.

2. The urinary catheter assembly of claim 1, wherein the arms are arranged in a first direction in the first, storage position, and in a second direction, being different from said first direction, in the second, use position.

3. The urinary catheter assembly of claim 1 or 2, wherein the arms, in the first, storage position, form a sealed compartment around said catheter shaft.

4. The urinary catheter assembly of claim 3, wherein at least one of the arms comprises a groove extending in the longitudinal direction of the arm, for accommodation of the catheter shaft in the first, storage position.

5. The urinary catheter assembly of claim 3 or 4, wherein the sealed compartment is gas and/or liquid impermeable.

6. The urinary catheter assembly of any one of the preceding claims, wherein the catheter shaft is provided with a hydrophilic surface and wherein the urinary catheter assembly further comprises a wetting fluid arranged with in the catheter assembly when the arms are in the first, storage position.

7. The urinary catheter assembly of any one of the preceding claims, wherein the arms when arranged in the second, use position are arranged at an angle in relation to the longitudinal direction of the shaft, said angle being in the range of 80-180 degrees, and preferably in the range of 85-140 degrees, and most preferably in the range of 90-120 degrees.

8. The urinary catheter assembly of any one of the preceding claims, wherein the arms when arranged in the second, use position are arranged at an angle in relation to the direction of the shaft, said angle being in the range of 80-120 degrees, and preferably in the range of 85-115 degrees, and most preferably in the range of 90-110 degrees.

9. The urinary catheter assembly of any one of the preceding claims, wherein the arms when arranged in the second, use position are arranged at an angle in relation to the direction of the shaft, said angle being in the range of 140-180 degrees, and preferably in the range of 150-180 degrees, and most preferably in the range of 160-180 degrees.

10. The urinary catheter assembly of any one of the preceding claims, wherein the arms are rotatable in opposite directions when rotated between the first, storage position and the second, use position.

11. The urinary catheter assembly of any one of the preceding claim, further comprising a valve, the valve closing the lumen when the arms are in the first, storage position, and the valve opening the lumen when the arms are in the second, use position.

12. The urinary catheter assembly of any one of the preceding claims, wherein each of the arms comprise a stub inserted into a corresponding hole in the rearward part of the catheter.

13. The urinary catheter assembly of any one of the preceding claims, wherein at least one of the arms is further provided with an additional lumen which is arranged to be in fluid communication with the lumen of the catheter shaft when the arms are arranged in the second, use position.

14. The urinary catheter assembly of any one of the preceding claims, wherein the rearward part of the catheter has an increased lateral dimension in at least one direction compared to the catheter shaft.
